# EUROPÄISCHE PATENTSCHRIFT

(11) **EP 1 615 864 B1**
(45) Veröffentlichungstag und Bekanntmachung des Hinweises auf die Patenterteilung: **02.07.2008**
(21) Anmeldenummer: 04727284.4
(22) Anmeldetag: 14.04.2004
(51) Int. Cl.: C07C 2/70, C07C 15/107, C07C 303/06, C07C 309/31

(54) **VERFAHREN ZUR HERSTELLUNG VON ALKYLAROMATISCHEN VERBINDUNGEN**
METHOD FOR PRODUCING ALKYL AROMATIC COMPOUNDS
PROCEDE DE PRODUCTION DE COMPOSES ALKYLAROMATIQUES

(30) Priorität: 15.04.2003 DE 10317294
(43) Veröffentlichungstag der Anmeldung: 18.01.2006
(73) Patentinhaber: BASF SE, 67056 Ludwigshafen (DE)
(72) Erfinder: STEINBRENNER, Ulrich, 67435 Neustadt (DE); NARBESHUBER, Thomas, 68165 Mannheim (DE); UNGER, Jörg, 67459 Böhl-Iggelheim (DE); ZEHNER, Peter, 67071 Ludwigshafen (DE); ZIMDAHL, Soeren, 69198 Schriesheim (DE); BENFER, Regina, 67122 Altrip (DE)
(74) Vertreter: Isenbruck, Günter
(86) Internationale Anmeldenummer: PCT/EP2004/003928
(87) Internationale Veröffentlichungsnummer: WO 2004/092096

(56) Entgegenhaltungen:
- WO-A-02/14266
- DE-A- 1 768 021
- DE-A- 2 413 444

## Beschreibung

Die Erfindung betrifft ein Verfahren zur Herstellung von alkylaromatischen Verbindungen durch Umsetzung von C₃₋₃₀-Olefinen oder Alkoholen, aus denen bei den Umsetzungsbedingungen C₃₋₃₀-Olefine gebildet werden, mit einem aromatischen Kohlenwasserstoff in Gegenwart eines Alkylierungskatalysators. Ferner betrifft die Erfindung ein Verfahren zur Herstellung von Alkylarylsulfonaten, bei dem die erhaltenen alkylaromatischen Verbindungen weiter umgesetzt werden.

Alkylbenzolsulfonate (ABS) werden seit langer Zeit als Tenside in Wasch- und Reinigungsmitteln eingesetzt. Nachdem zunächst derartige Tenside auf Basis von Tetrapropylen eingesetzt wurden, die jedoch schlecht biologisch abbaubar waren, wurden in der Folgezeit möglichst lineare Alkylbenzolsulfonate (LAS) hergestellt und verwendet. Lineare Alkylbenzolsulfonate weisen jedoch nicht in allen Anwendungsgebieten ausreichende Eigenschaftsprofile auf.

Daher wurden teilweise verzweigte Alkylbenzolsulfonate entwickelt, die ein verbessertes Eigenschaftsprofil zeigen. Sie zeigen insbesondere gute Kaltwascheigenschaften und Verträglichkeit mit hartem Wasser. In der WO 02/14266 sind derartige Alkylarylsulfonate und Verfahren zu ihrer Herstellung beschrieben. Die Herstellung erfolgt durch Umsetzung eines C₄-Olefin-Gemisches an einem Metathesekatalysator, Dimerisierung der aus der Metathese erhaltenen C_{5/6}-Olefine, Alkylierung von aromatischen Kohlenwasserstoffen mit den so erhaltenen C₁₀₋₁₂-Olefinen, Sulfonierung der alkylaromatischen Verbindungen und gegebenenfalls Neutralisierung. Das Verfahren erlaubt es, einen geeigneten Verzweigungsgrad in den Alkylresten einzustellen. Zu starke Verzweigungen benachteiligen die biologische Abbaubarkeit der Produkte, während zu lineare Produkte die Viskosität und die Löslichkeit der Sulfonate negativ beeinflussen.

Auch die WO 02/44114 beschreibt Verfahren zur Herstellung von Alkylarylsulfonaten. Die dazu erforderlichen teilweise verzweigten Alkylarylverbindungen werden durch Alkylierung von aromatischen Kohlenwasserstoffen mit speziellen Olefingemischen erhalten. Die Alkylierung wird dabei in Gegenwart eines Zeoliths des Typs Faujasit als Alkylierungskatalysator durchgeführt. DE-1 768 021 betrifft Verfahren zur Herstellung alkylaromatischer Verbindungen.

In den Alkylierungsverfahren erfolgt die Umsetzung in der Regel in einem einzigen Reaktor in kontinuierlicher oder diskontinuierlicher Fahrweise. Aromatischer Kohlenwasserstoff und Olefin werden dabei an einer Stelle in den Reaktor eingespeist. Diese Vorgehensweise führt nicht immer zu ausreichenden Standzeiten des Katalysators. Insbesondere die Aktivität des Katalysators kann über längere Zeiträume abnehmen.

Aufgabe der vorliegenden Erfindung ist die Bereitstellung eines Verfahrens zur Alkylierung von aromatischen Kohlenwasserstoffen, das die Nachteile der bestehenden Verfahren vermeidet und insbesondere die Katalysatorstandzeit erhöht.

Die Aufgabe wird erfindungsgemäß gelöst durch ein Verfahren zur Herstellung von alkylaromatischen Verbindungen durch Umsetzung von C₃₋₃₀-Olefinen oder Alkoholen, aus denen bei den Umsetzungsbedingungen C₃₋₃₀-Olefine gebildet werden, mit einem aromatischen Kohlenwasserstoff in Gegenwart eines Alkylierungskatalysators, wobei die Umsetzung in einer Reaktorkaskade aus mindestens zwei Reaktoren durchgeführt wird, wobei jeder der Reaktoren den Alkylierungskatalysator enthält, mindestens 80 % des aromatischen Kohlenwasserstoffs in den ersten Reaktor der Reaktorkaskade eingespeist werden und mindestens 40 % der Olefine nach dem ersten Reaktor zwischeneingespeist werden.

Es wurde erfindungsgemäß gefunden, dass bei einer Umsetzung in einer Reaktorkaskade mit Zwischeneinspeisung der Olefine die Katalysatorstandzeiten erheblich verbessert werden können. Bis zu einer Regenerierung des Katalysators kann dadurch mehr Produkt hergestellt werden.

Erfindungsgemäß ist die Reaktorkaskade aus mindestens zwei hintereinander geschalteten Reaktoren aufgebaut. Vorzugsweise werden mindestens drei Reaktoren eingesetzt. Die Höchstzahl der Reaktoren ist dabei nur durch die praktischen Gegebenheiten begrenzt. Vorzugsweise beträgt die Anzahl an Reaktoren 3 bis 20, besonders bevorzugt 3 bis 10.

Die Reaktoren sind dabei hintereinander geschaltet, so dass der Austrag aus dem ersten Reaktor in den zweiten Reaktor eingeführt wird. Entsprechendes gilt für die weiteren Reaktoren.

Die Reaktoren weisen dabei vorzugsweise eine Rührkesselcharakteristik auf. Dies bedeutet, dass in den Reaktoren eine Umwälzung der eingetragenen Stoffe stattfindet, die vorzugsweise mindestens das Zweifache, besonders bevorzugt mindestens das Dreifache, insbesondere mindestens das Fünffache des Feedstroms beträgt. Es kann sich damit beispielsweise bei den einzelnen Reaktoren um Rührkessel, Schlaufenreaktoren, Reaktoren mit externem Umlauf, Strahlschlaufenreaktoren sowie Reaktoren mit Fliess- oder Wanderbetten handeln.

Im erfindungsgemäßen Verfahren werden mindestens 80 % des aromatischen Kohlenwasserstoffs in den ersten Reaktor der Reaktorkaskade eingespeist. Vorzugsweise werden mindestens 90 % des aromatischen Kohlenwasserstoffs in den ersten Reaktor eingespeist, besonders bevorzugt im wesentlichen die gesamten aromatischen Kohlenwasserstoffe bzw. die gesamten aromatischen Kohlenwasserstoffe.

Mindestens 40 % der Olefine werden nach dem ersten Reaktor zwischeneingespeist. Entsprechend werden maximal 60 % der Olefine in den ersten Reaktor der Reaktorkaskade eingespeist. Die Zwischeneinspeisung erfolgt abgesehen vom ersten Reaktor der Reaktorkaskade in mindestens einem weiteren Reaktor der Reaktorkaskade. Besonders bevorzugt erfolgt eine Zwischeneinspeisung vor jedem der Reaktoren der Reaktorkaskade. Die Anteile des Olefins, die vor jedem Reaktor bzw. in jedem Reaktor zwischeneingespeist werden, können frei gewählt werden. Vorzugsweise unterscheiden sich die Einspeisungsströme jeweils maximal um 50 %. Besonders bevorzugt werden in etwa gleiche Anteile des Olefins in jeden Reaktor (zwischen)eingespeist. Insbesondere werden jeweils gleiche Anteile des Olefins in jeden Reaktor (zwischen)eingespeist. Bevorzugt wird das Olefin bei der Einspeisung gut eingerührt oder eingemischt. Dies wird beispielsweise durch aktive oder passive Elemente bewerkstelligt, beispielsweise durch Pumpen, statische Mischer oder Inertbetten. Bevorzugt wird der Feed in oder vor die Umwälzpumpe(n) eindosiert.

Erfindungsgemäß enthält jeder der Reaktoren den Alkylierungskatalysator. Vorzugsweise unterscheiden sich die Katalysatormengen in den einzelnen Reaktoren um maximal 50%, besonders bevorzugt maximal 20%, insbesondere maximal 10%, bezogen auf den Reaktor mit der größten Menge an Katalysator. Dies bedeutet, dass jeweils die Differenz zwischen der Menge des Katalysators im Reaktor mit der größten Katalysatormenge und jeder der anderen Reaktoren, geteilt durch die Menge des Katalysators im Reaktor mit der größten Katalysatormenge, maximal 50%, besonders bevorzugt maximal 20%, insbesondere maximal 10% beträgt. Insbesondere bevorzugt enthalten alle Reaktoren die gleiche Menge an Katalysator.

Das erfindungsgemäße Verfahren führt dazu, dass man - gemittelt über die gesamte Katalysatormenge - verbesserte Katalysatorstandzeiten und damit mehr Produkt bis zu einer Regenerierung des Katalysators erhält.

Die Art der Zwischeneinspeisung ist dabei frei wählbar. Die Reaktoren sind typischerweise mit Verbindungsrohren miteinander verbunden. In diese Verbindungen können Pumpen und Abzweigungen integriert sein, um die Reihenfolge der Verschaltung der Reaktoren verändern zu können. Die Olefin-Zwischeneinspeisungen können nun unabhängig von der Verbindung der einzelnen Reaktoren untereinander in einem getrennten Zustrom jeweils in den Reaktor erfolgen, oder die Zwischeneinspeisungen können vor dem jeweiligen Reaktor erfolgen, so dass nur durch ein Rohr das umzusetzende Gemisch in den Reaktor eingetragen wird. Entsprechende Geometrien sind dem Fachmann bekannt.

Die apparative Steuerung der Olefineinspeisung erfolgt in an sich bekannter Weise durch einzelne Pumpen, Ventile, Düsen, Blenden, Spalten oder andere geeignete Vorrichtungen.

Gemäß der vorliegenden Erfindung ist es nicht unbedingt erforderlich, dass jeder Reaktor, in dem sich eine Zugabestelle für das Olefin befindet, als Einzelaggregat wie z.B. als Rührkessel ausgebildet ist. Es können auch konstruktive Ausgestaltungen eines Reaktors eingesetzt werden, die die Funktion einer Serienschaltung mehrerer Reaktorelemente erfüllen. Es kann daher auch ein Einzelreaktor, insbesondere ein Festbettreaktor, eingesetzt werden, der durch geeignete Einbauten wie Lochblenden oder Siebböden in mindestens zwei, vorzugsweise mindestens drei Segmente unterteilt ist. Weiterhin ist es möglich, Rührkolonnen mit mehr als einer Stufe und Strömungsrohre jeweils mit mehreren Zugabestellen einzusetzen.

Die Steuerung der Zugabe der Olefine kann an die praktischen Erfordernisse angepasst werden. Beispielsweise kann die Menge des zugebenen Olefins an die im jeweiligen Reaktor vorliegende Menge des Katalysators und an den jeweiligen Katalysatorzustand (Deaktivierung) angepasst werden. Die Zugabe wird bevorzugt so gesteuert, dass in jedem Reaktor, bezogen auf die Katalysatormenge, die gleiche inkrementelle Produktivität geleistet wird. Die kontinuierlichen Steuerungen der Zugaben der Olefine können an den jeweiligen Umsatz im Reaktor angepasst werden. Demgemäss wird gemäß einer Ausführungsform der Erfindung eine Zwischeneinspeisung des Olefins vor jedem der Reaktoren durchgeführt, und die jeweils zwischeneingespeiste Olefinmenge wird so gesteuert, dass in jedem Reaktor, bezogen auf die jeweilige Katalysatormenge, die gleiche inkrementelle Produktivität erreicht wird. Hierdurch wird sichergestellt, dass die jeweils zugegebene Olefinmenge an die in der jeweiligen Stufe bzw. im jeweiligen Reaktor vorliegende Katalysatormenge angepasst wird, sofern der Katalysator nicht auf alle Stufen bzw. Reaktoren gleich verteilt ist. Die zugegebene Olefinmenge wird vorzugsweise so eingestellt, dass in jedem Reaktor, bezogen auf die jeweilige Katalysatormenge, die gleiche Zunahme an Umsetzungsprodukt erreicht wird. Dies wird durch den Ausdruck "gleiche inkrementelle Produktivität" umschrieben. Anders ausgedrückt wird, bezogen auf die jeweilige Katalysatormenge, in jedem Reaktor die Produktausbeute gleichmäßig erhöht, so dass am jeweiligen Katalysator pro Zeiteinheit gleich viele Umsetzungen stattfinden.

Sofern die in den unterschiedlichen Reaktoren vorliegenden Katalysatoren einen unterschiedlichen Deaktivierungsgrad aufweisen, kann dies durch Steuerung der Olefin-Einspeisungsmenge entsprechend kompensiert werden.

Wenn sich die Katalysatormengen in den Reaktoren um vorzugsweise maximal 50%, besonders bevorzugt maximal 20%, insbesondere maximal 10%, bezogen auf den Reaktor mit der größten Menge an Katalysator, unterscheiden, gilt dies entsprechend für die Olefineinspeisung, sofern die Katalysatoren in den Reaktoren den gleichen Deaktivierungsgrad aufweisen. Entsprechend beträgt die Abweichung der kleinsten Olefinbelastung in einem Reaktor von der größten Olefinbelastung in einem Reaktor maximal 50%, besonders bevorzugt maximal 20%, insbesondere maximal 10% der größten Olefinbelastung. Die Zugabemengen der Olefin-Zwischeneinspeisung werden damit an die jeweils vorliegenden Katalysatormengen angepasst.

Besonders bevorzugt werden erfindungsgemäß Katalysator und Olefin gleichmäßig auf alle Reaktoren verteilt bzw. in alle Reaktoren (zwischen)eingespeist. Dennoch sind die vorstehend genannten Abweichungen möglich.

Im folgenden sind unterschiedliche unabhängige bevorzugte Ausführungsformen des erfindungsgemäßen Verfahrens beschrieben, wobei von einer Reaktorkaskade aus n Reaktoren ausgegangen wird. Der erste Reaktor, in dem die Umsetzung beginnt, wird dabei als Reaktor 1 bezeichnet. Höhere Zahlen bezeichnen Reaktoren, die weiter stromabwärts in der Reaktorkaskade vorgesehen sind.

Um eine gleichmäßige Deaktivierung aller Reaktoren zu gewährleisten, kann nach einer Zeit, die idealerweise 1/n der Standzeit beträgt, die Kaskade um einen Reaktor weitergeschaltet werden, d.h. Reaktor 1 tritt an Stelle von Reaktor 2, Reaktor 2 an Stelle von Reaktor 3, ...., Reaktor n-1 an Stelle von Reaktor n und Reaktor n an Stelle von Reaktor 1. Selbstverständlich sind andere Permutationen denkbar - z.B. bei 5 Reaktoren 1 ⇒ 3, 2 ⇒ 4, 3 ⇒ 5,4 ⇒ 1, 5 ⇒ 2 -, die dazu führen, dass sich jedes Katalysatorbett etwa die gleiche Zeit an jeder Position in der Kaskade befunden hat.

Erfindungsgemäß kann damit die Reihenfolge der Reaktoren innerhalb der Kaskade in zeitlichen Abständen so geändert werden, dass jeder Reaktor jede der Positionen innerhalb der Kaskade für den gleichen Zeitraum einnimmt. Abweichungen von bis zu 25 %, vorzugsweise bis zu 10 %, insbesondere bis 5 % von dieser Vorgabe sollen innerhalb des Ausdrucks "gleicher Zeitraum" liegen.

Auch sind nur unvollständige Permutationen möglich, z.B. indem 1 mit n, 2 mit n-1 u.s.w. tauscht. Ebenfalls kann der Tausch nicht nur nach einer festen Zeit, sondern auch mit zunehmender Deaktivierung des Katalysators rascher oder später erfolgen.

Da sich zeigte, dass bei kaskaden- oder serienförmiger Verschaltung der Katalysator in Reaktor 1 am wenigsten, der in Reaktor n am stärksten deaktiviert wurde, kann alternativ in Reaktor 1 weniger Katalysator als in Reaktor 2, in Reaktor 2 weniger Katalysator als in Reaktor 3 u.s.w. eingefüllt werden, um eine einigermaßen gleichförmige Deaktivierung aller Betten zu erzielen.

Es kann auf eine gleichförmige Deaktivierung auch verzichtet werden. Aus Gründen der Effizienz beim Katalysatorwechsel oder bei der Katalysatorregeneration einer Großanlage ist es oft vorteilhaft, wenn nicht die gesamte Katalysatormasse auf einmal ausgetauscht bzw. regeneriert werden muss. In diesem Fall könnte man z.B. die Umschaltung der Reaktoren unterlassen und den Katalysator in Reaktor n öfter, den Katalysator in Reaktor 1 seltener wechseln oder regenerieren. Hier kann man zweckmäßigerweise den Umsatz nach jedem Reaktor bestimmen. Weicht dieser zu stark nach unten ab, so muss der Katalysator in diesem Reaktor gewechselt werden. Die Grenze liegt typischerweise zwischen 95% und 98% Umsatz.

Ob und wie die Reaktoren im Betrieb neu verschaltet werden, in welchem Verhältnis die Katalysatormasse und Olefin-Zwischeneinspeisungen auf die einzelnen Reaktoren verteilt werden, richtet sich nach den praktischen Erfordernissen. Das hier günstigste Optimum kann mit einer geeigneten Versuchsplanung leicht für einen definierten Katalysator und ein definiertes Olefin ermittelt werden.

Geeignete Olefine sind lineare, cyclische und verzweigte Monoolefine mit 3 bis 30 C-Atomen, z.B. Propylen, 1-Buten, 2-Buten, Isobuten, 1-Penten, 2-Penten, 3-Penten, Cyclopenten, die Methylbutene, die n-Hexene, die Methylpentene, Cyclohexen, Methylcyclohexen, Decene, Undecene, Dodecene, usw.. Bevorzugt sind lineare und verzweigte C₁₀-C₁₄-Monoolefine, besonders bevorzugt C₁₀-C₁₂-Monoolefine, die 0 bis 3 Methyl- und/oder E-thylverzweigungen in der Seitenkette aufweisen. Das Olefin stammt bevorzugt aus den in WO 02/14266 oder WO 02/44114 genannten Quellen.

Beispielsweise können C₁₀₋₁₂-Olefine eingesetzt werden, die einen mittleren Verzweigungsgrad im Bereich von 1 bis 2,5, besonders bevorzugt 1 bis 2,0, insbesondere 1 bis 1,5 und speziell 1 bis 1,2 aufweisen. Als Verzweigungsgrad eines reinen Olefins ist dabei die Zahl der Kohlenstoffatome, die mit drei Kohlenstoffatomen verknüpft sind, plus zwei mal die Zahl der Kohlenstoffatome, die mit 4 Kohlenstoffatomen verknüpft sind, definiert. Der Verzweigungsgrad eines Rein-Olefins ist dabei leicht nach Totalhydrierung zum Alkan via ¹H-NMR über die Integration der Signale der Methylgruppen relativ zu den Methylen- und Methinprotonen messbar.

Bei Mischungen von Olefinen werden die Verzweigungsgrade mit den Molprozenten gewichtet, und so wird ein mittlerer Verzweigungsgrad errechnet.

Die molaren Anteile bestimmt man dabei optimalerweise mittels Gaschromatographie.

Die Art der Verzweigungen im Olefin ist dabei bevorzugt so gestaltet, dass nach Hydrierung weniger als 10%, bevorzugt weniger als 5%, besonders bevorzugt weniger als 1% Alkane erhalten werden, die nicht zu den Methyl-, Dimethyl-, Ethylmethyl- und Diethylalkanen zählen. Dies bedeutet, dass die Verzweigungen nur Methyl- und Ethyl-Verzweigungen sind.

Die Olefine können erhalten werden durch Umsetzung eines C₄-Olefin-Gemisches an einem Metathesekatalysator zur Herstellung eines 2-Penten und/oder 3-Hexen enthaltenden Olefingemisches und gegebenenfalls Abtrennung von 2-Penten und/oder 3-Hexen,

gefolgt von einer Dimerisierung des so erhaltenen 2-Pentens und/oder 3-Hexens in Gegenwart eines Dimerisierungskatalysators zu einem C₁₀₋₁₂-Olefin enthaltenden Gemisch, Abtrennung der C₁₀₋₁₂-Olefine und gegebenenfalls Abtrennung von 5 bis 30 Gew.-%, bezogen auf die abgetrennten C₁₀₋₁₂-Olefine, an Leichtsiederbestandteilen der C₁₀₋₁₂-Olefine.

Zur Herstellung eines gewünschten Verzweigungsgrades ist es möglich, den Olefinen lineare Olefine beizumischen oder einen Teil der stark verzweigten Olefine abzutrennen. Bei einer Zumischung können beispielsweise 5 bis 60 Gew.-% an linearen Olefinen zugesetzt werden.

Der Metathesekatalysator in der Metathese ist vorzugsweise ausgewählt aus Verbindungen eines Metalls der Nebengruppen VIb, VIIb oder VIIIb des Periodensystems der Elemente.

Als Dimerisierungskatalysator wird vorzugsweise ein Katalysator eingesetzt, der wenigstens ein Element der VIII. Nebengruppe des Periodensystems der Elemente enthält.

Das in der Metathese eingesetzte C₄-Olefin-Gemisch kann aus Steamcracker- oder Raffinerie-C₄-Strömen stammen. Aus diesen Strömen wird dabei zunächst Butadien zusammen mit acetylenischen Verunreinigungen durch Extraktion und/oder Selektivhydrierung abgetrennt. Sodann kann eine Abtrennung von Isobuten durch Umsetzung mit einem Alkohol in Gegenwart eines sauren Katalysators zu einem Ether erfolgen. Der Ether und der Alkohol können sodann abgetrennt werden. Anschließend können Oxygenat-Verunreinigungen aus dem Austrag der vorstehenden Schritte an entsprechend ausgewählten Adsorbermaterialien abgetrennt werden. Es schließt sich daraufhin die Metathesereaktion, wie sie beschrieben ist, an.

Für weitere Einzelheiten der jeweiligen Schritte kann auf die WO 02/14266 sowie WO 00/39058 verwiesen werden.

Die erhaltenen C₁₀₋₁₂-Olefin-Gemische weisen eine optimale Struktur/Linearität auf. Dies bedeutet, dass der Verzweigungsgrad und die Art der Verzweigung optimal gewählt sind, um bei der Alkylierung vorteilhafte alkylaromatische Verbindungen zu erhalten. Die Einstellung der optimal einzusetzenden C₁₀₋₁₂-Olefin-Gemische kann beispielsweise durch Zumischen linearer Olefine erfolgen. Besonders bevorzugt wird bei der Dimerisierung ein geeigneter Katalysator mit einer geeigneten Verfahrensweise kombiniert, um zum optimalen C₁₀₋₁₂-Olefin-Gemisch zu gelangen. Bei dieser Verfahrensweise werden bei der Alkylierung direkt die gewünschten Strukturen erhalten. Man kann in diesem Fall auf das Zumischen linearer Olefine und das Abtrennung höher verzweigter Olefine verzichten. Es sind auch Kombinationen der beschriebenen Verfahrensweisen möglich.

Alternativ können die zur Alkylierung eingesetzten Olefine oder Alkohole wie folgt erhalten werden:

Ein Kohlenwasserstoffgemisch wird bereitgestellt, das im Wesentlichen Monoolefine mit 4-6 Kohlenstoffatomen enthält. Sodann wird das Monoolefingemisch durch Umsetzung mit Kohlenmonoxid und Wasserstoff in Gegenwart eines Hydroformylierungskatalysators unter Erhalt eines Aldehydgemisches hydroformyliert. Das Aldehydgemisch wird dann einer Aldolkondensation unter Erhalt eines Gemisches kondensierter α,β-ungesättiger Aldehyde unterzogen. Das so erhaltene Gemisch wird dann mit Wasserstoff in Gegenwart eines Hydrierkatalysators unter Erhalt eines Gemisches gesättigter Alkohole mit 10 bis 14 Kohlenstoffatomen hydriert. Dieses Alkoholgemisch kann direkt zur Umsetzung mit dem aromatischen Kohlenwasserstoff in Gegenwart eines Alkylierungskatalysators eingesetzt werden. Alternativ kann das erhaltene Alkoholgemisch zu einem Olefingemisch dehydratisiert werden, das im Wesentlichen Olefine mit 10 bis 14 Kohlenstoffatomen enthält.

Das ursprünglich eingesetzte olefinhaltige Kohlenwasserstoffgemisch kann aus einer Metathese von 1-Buten und 2-Buten abgeleitet sein.

Die zur Alkylierung eingesetzten Olefine können auch durch Extraktion vorwiegend einfach verzweigter Paraffine aus Kerosinschnitten und nachfolgende Dehydrierung, durch Fischer-Tropsch-Synthese von Olefinen oder Paraffinen, wobei die Paraffine dehydriert werden, durch Dimerisierung kürzerkettiger interner Olefine oder durch Isomerisierung von linearen Olefinen oder Paraffinen erhalten werden, wobei die isomerisierten Paraffine dehydriert werden. Entsprechende Verfahrensweisen sind in der WO 02/44114 beschrieben.

Für die Sequenz aus Metathese und Dimerisierung kann auch auf die DE-A-199 32 060 verwiesen werden.

Alternativ zu Olefinen können auch Alkohole in die Reaktion eingeschleust werden, da diese unter Bedingungen der Aromatenalkylierung rasch H₂O abspalten und Olefine bilden. Alkohole sind demnach mit den aus ihnen durch Dehydratisierung gebildeten Olefinen gleichzusetzen.

Geeignete Aromaten sind beispielsweise Benzol, Toluol, Ethylbenzol und die Xylole, bevorzugt sind Benzol, Toluol und Ethylbenzol, besonders bevorzugt ist Benzol.

Geeignete Katalysatoren sind heterogene Säuren, wie z.B. Tone, insbesondere Montmorillonit oder montmorrillonithaltige Materialien wie z.B. K10 und K20 von Südchemie, stark saure Ionentauscher wie z.B. Amberlyst^{®}36 oder Amberlyst^{®}15 von Rohm und Haas oder Nafion^{®} bzw. Nafion^{®}/Silica von DuPont, saure Metalloxide wie z.B. M(I)O₃-M(II)O₂-M(I)=W, Mo und M(II) = Zr, Ti, Mn und/oder Sn, Al₂O₃-SiO₂, TiO₂-ZrO₂, TiO₂, Nb₂O₅, Ta₂O₅, sulfatisierte Metalloxide wie z.B. ZrO₂-SO₃, TiO₂-SO₃, Al₂O₃-SO₃, WO₃-SO₃, Nb₂O₅-SO₃, geträgerte Heteropolysäuren wie z.B. PW₁₂-HPA/SiO₂, PMo₁₂-HPA/Kohle, P₂W₁₈-HPA/TiO₂ und Zeolithe.

Bevorzugt sind Zeolithe der Strukturtypen BIK; BRE, ERI, CHA, DAC, EAB, EDI, EPI, FER, Pentasile mit MFI- oder MEL-Struktur, Faujasite wie z.B. Y, LTL, MOR, BEA, GME, HEU, KFI, MAZ, OFF, PAU, RHO, STI. Besonders bevorzugt sind L, Y inklusive den USY-Typen, BEA und MOR.

Diese Zeolithe werden bevorzugt in der H- und/oder La-Form eingesetzt, jedoch können je nach Herstellung Spuren an Na, K, Mg oder Ca anwesend sein. Ein teilweiser oder vollständiger Austausch des Gitter-Aluminium durch B, Ga oder Fe ist möglich.

Der Katalysator kann z.B. als feines Pulver in Suspension direkt verwendet werden, bei Zeolithen sind dies z.B. Teilchengrößen zwischen 100 nm und einigen µm. Meist werden diese Katalysatoren jedoch zusammen mit Bindermaterialien zu Formkörpern von 0,1-5 mm Durchmesser verformt. Zum Einsatz in Festbetten sind 1-3 mm bevorzugt, in Suspension 0,001-1 mm, in Fließbetten 0,1-3 mm. Als Binder eignen sich besonders Tone, Aluminiumoxide wie z.B. Purale, Sirale und Versale und Kieselgele. Weiter können inerte Füllstoffe wie SiO₂ (z.B. Aerosil von Degussa) zugesetzt werden.

Als Formkörper eignen sich z.B. Tabletten, Stränglinge, Ringe, Rippstränge, Stern- oder Wagenradextrudate.

Die Katalysatoren besitzen vorzugsweise spezifische Oberflächen von 30 bis 2000 m²/g, bevorzugt 100 bis 700 m²/g. Das Volumen der Poren mit Durchmesser 2-20 nm beträgt typischerweise 0,05-0,5 ml/g, bevorzugt 0,1-0,3 ml/g, das der Poren von 20-200 nm typischerweise 0,005 bis 0,2 ml/g, bevorzugt 0,01 bis 0,1 ml/g, das der Poren von 200-2000 nm typischerweise 0,05-0,5 ml/g, bevorzugt 0,05 bis 0,3 ml/g.

Deaktivierte Katalysatoren können in den meisten Fällen durch Abbrennen in Luft oder Magerluft bei 250-550 °C regeneriert werden. Alternativ ist eine Behandlung mit bei tieferer Temperatur - optional auch in der Flüssigphase - oxidierend wirkenden Verbindungen möglich, hier sind insbesondere NOₓ, H₂O₂ und die Halogene zu nennen. Die Regenerierung kann direkt im Alkylierungsreaktor oder extern erfolgen.

Die Alkylierung findet bevorzugt in der Flüssigphase, d.h. ohne Gasphase statt, was durch einen entsprechenden Systemdruck erreicht werden kann. In der Regel arbeitet man bei Eigendruck (dem Dampfdruck des Systems) oder darüber. Alkylierungstemperaturen sind bevorzugt 100 bis 250 °C, besonders bevorzugt 120 bis 220 °C, ganz besonders bevorzugt 130 bis 200 °C. Geeignete Drücke liegen beispielsweise im Bereich von 1 bis 35 bar.

Bei der Auswahl des Katalysators sollte darauf geachtet werden, dass die Bildung von Verbindungen minimiert wird, die im Alkylrest Kohlenstoffatome mit einem H/C-Index von 0 beinhalten. Der H/C-Index definiert dabei die Anzahl an Wasserstoffatomen pro Kohlenstoffatom im Alkylrest. Die erfindungsgemäß erhaltenen alkylaromatischen Verbindungen weisen einen charakteristischen Anteil von primären, sekundären, tertiären und quartären Kohlenstoffatomen im Alkylrest (Seitenkette) auf. Es sollen insbesondere Verbindungen gebildet werden, die im Mittel im Alkylrest ein bis drei Kohlenstoffatome mit einem H/C-Index von eins aufweisen. Dies kann insbesondere durch die Auswahl geeigneter Katalysatoren erreicht werden, die einerseits durch ihre Geometrie die Bildung der unerwünschten Produkte unterdrücken und andererseits aber eine ausreichende Reaktionsgeschwindigkeit zulassen.

Vorzugsweise weisen die erfindungsgemäßen Gemische alkylaromatischer Verbindungen nur eine geringen Anteil an C-Atomen im Alkylrest mit einem H/C-Index von Null auf. Vorzugsweise ist der Anteil an Kohlenstoffatomen im Alkylrest mit einem H/C-Index von Null im Mittel aller Verbindungen <15 %, besonders bevorzugt <10%. Der Anteil an Kohlenstoffatomen, im Alkylrest mit einem H/C-Index von Null, die gleichzeitig an den Aromaten gebunden sind, beträgt vorzugsweise mindestens 80%, besonders bevorzugt mindestens 90%, insbesondere mindestens 95% aller Kohlenstoffatome im Alkylrest mit einem H/C-Index von Null.

Vorzugsweise weisen die erfindungsgemäß erhaltenen Gemische alkylaromatische Verbindungen im Mittel 1 bis 3, bevorzugt 1 bis 2,5, besonders bevorzugt 1 bis 2 Kohlenstoffatome in der Seitenkette (d.h. ohne die aromatischen Kohlenstoffatome zu zählen) mit einem H/C-Index von 1 auf. Der Anteil an Verbindungen mit drei Kohlenstoffatomen dieses Typs liegt bevorzugt bei weniger als 30%, besonders bevorzugt bei weniger als 20%, insbesondere bei weniger als 10%.

Eine Steuerung des Anteils der Kohlenstoffatome, die einen bestimmten H/C-Index aufweisen, kann durch geeignete Wahl des eingesetzten Katalysators erfolgen. Bevorzugt eingesetzte Katalysatoren, mit denen vorteilhafte H/C-Verteilungen erzielt werden, sind Mordenit, Beta-Zeolith, L-Zeolith und Faujasite. Insbesondere bevorzugt sind Mordenit und Fauj asite.

Bei der Umsetzung beträgt das globale - d.h. über alle Reaktoren gerechnete - Verhältnis von Aromat: Olefin üblicherweise zwischen 1:1 und 100:1 bevorzugt 2:1 bis 20:1 (Molverhältnis). Das globale Verhältnis bezeichnet dabei das Verhältnis der Summe aller Aromatströme in die Kaskade zur Summe aller Olefinströme in die Kaskade.

Die erhaltenen alkylaromatischen Verbindungen können sodann sulfoniert und zu Alkylarylsulfonaten neutralisiert werden. Die Erfindung betrifft daher auch ein Verfahren zur Herstellung von alkylaromatischen Verbindungen durch Umsetzung von C₃₋₃₀-Olefinen oder Alkoholen, aus denen bei den Umsetzungsbedingungen C₃₋₃₀-Olefine gebildet werden, mit einem aromatischen Kohlenwasserstoff in Gegenwart eines Alkylierungskatalysators, wobei die Umsetzung in einer Reaktorkaskade aus mindestens zwei Reaktoren durchgeführt wird, wobei jeder der Reaktoren den Alkylierungskatalysator enthält, mindestens 80 % des aromatischen Kohlenwasserstoffs in den ersten Reaktor der Reaktorkaskade eingespeist werden und mindestens 40 % der Olefine nach dem ersten Reaktor zwischeneingespeist werden.

Die Alkylaryle werden beispielsweise durch Sulfonierung (z.B. mit SO₃ , Oleum, Chlorsulfonsäure, bevorzugt mit SO₃) und Neutralisation (z.B. mit Na-, K, NH₄-, Mg-Verbindungen, bevorzugt mit Na-Verbindungen) zu Alkylarylsulfonaten umgesetzt. Sulfonierung und Neutralisation sind in der Literatur hinreichend beschrieben und werden nachdem Stand der Technik ausgeführt. Die Sulfonierung wird bevorzugt in einem Fallfilmreaktor ausgeführt, kann aber auch in einem Rührkessel erfolgen. Die Sulfonierung mit SO₃ ist der Sulfonierung mit Oleum vorzuziehen.

Die nachdem beschriebenen Verfahren hergestellten Verbindungen können zur Herstellung von Wasch- und Reinigungsmitteln eingesetzt werden, wie es in der WO 02/14266 beschrieben ist. In dieser Literaturstelle sind auch weitere Inhaltsstoffe für Wasch- und Reinigungsmittel genannt.

Die Erfindung wird durch die nachstehenden Beispiele erläutert:

### Beispiele

### Herstellung des Katalysators:

4000 g Mordenit FM-8 (von Uetikon, Si : Al =12,2 mol/mol) wurden mit 2670 g Pural® SB (von Sasol), 133g HCOOH und 3500 g dest H₂O in einem Laborkneter 45 Minuten verdichtet und zur Stränglingen von 1,5 mm Durchmesser extrudiert. Diese wurden 16h bei 500°C unter Luft kalziniert und auf eine Siebfraktion von 0,3 - 0,5 mm gesplittet.

Der Katalysator besaß eine spezifische Oberfläche nach Langumir von 519 +- 5m²/g - errechnet aus der 5-Punkt N₂-Isotherme gemäß DIN 66131.

### Aufbau der Reaktoren:

Jeder Reaktor bestand aus einem gewendelten V2A-Strahlrohr mit 200 ml Innenvolumen, einer Siebplatte und einem 5µm-Filter am Ausgang. Der Flüssigumlauf wurde durch eine HPLC-Pumpe (von Kontron) bewerkstelligt. Das Verhältnis Umlauf : Zulauf betrug stets ca. 10 : 1. Die Reaktoren wurden durch einen Umluftofen beheizt. Die Katalysatorschüttung von 70g befand sich zwischen zwei Inertschüttungen aus Quarzglaskugeln von je ca. 20 g. Jeder Reaktor arbeitete bei einem Systemdruck von 30 bar, welcher von den Feedpumpen (HPLC-Pumpen von Kontron) aufgebracht und von einem Überströmer gehalten wurde.

### Versuchsdurchführung:

Vor Versuchsbeginn wird der in den Reaktoren eingebaute Katalysatorsplitt 5 h bei 500°C mit synthetischer Luft frisch aktiviert, dann wird abgekühlt und jeder Reaktor mit reinem Benzol gefüllt. Anschließend wird auf 160°C aufgeheizt, und Umläufe und Feed werden gestartet. Die Feedströme werden durch ein bei Raumtemperatur gehaltenes Bett an Katalysator und 3A-Molsieb gereinigt.

Das verwendete Dodecen entstammt der nickelkatalysierten Dimersierung von 3-Hexen nach den nachstehenden Varianten a) oder b):

### Variante a)

Eine butadienfreie C₄-Fraktion mit einem Gesamtbutengehalt von 84,2 Gew.-% sowie einem Molverhältnis 1-Buten zu 2-Butene von 1 zu 1,06 wird bei 40°C und 10 bar kontinuierlich über einen mit Re₂O₇/Al₂O₃-Heterogenkontakt bestückten Rohrreaktor geleitet. Die Katalysator-Belastung beträgt im Beispiel 4500 kg/m²h. Der Reaktionsaustrag wird destillativ getrennt und enthält folgende Komponenten (Angaben in Massenprozent):

Ethen 1,15 %; Propen 18,9 %, Butane 15,8 %, 2-Butene 19,7 %, 1-Buten 13,3 %, i-Buten 1,0 %, 2-Penten 19,4 %, Methylbutene 0,45 %, 3-Hexen 10,3 %.

2-Penten und 3-Hexen werden aus dem Produkt destillativ in Reinheiten > 99 Gew.-% gewonnen.

### Variante b)

### Kontinuierliche Dimerisierung von 3-Hexen im Festbettverfahren

- Katalysator:: 50 % NiO, 34 % SiO₂, 13 % TiO₂, 3 % Al₂O₃ (gemäß DE 43 39 713) eingesetzt als 1-1,5 mm Splitt (100 ml), 24 h bei 160°C in N₂ konditioniert
- Reaktor:: isotherm, 16 mm-∅-Reaktor
- WHSV:: 0,25 kg/l.h
- Druck:: 20 bis 25 bar
- Temperatur:: 100 bis 160°C

| | | | | | | | | |
|---|---|---|---|---|---|---|---|---|
| Temperatur | | 100 | 120 | 140 | 160 | 160 | | |
| (°C) | | | | | | | | |
| Druck | **Feed-** | 20 | 20 | 20 | 25 | 25 | Sammel | **C₁₂-** |
| (bar) | **stock** | | | | | | - | **Destillat** |
| Betriebsstunden | | 12 | 19 | 36 | 60 | 107 | Produkt | |
| Flüssiganfall | | 24 | 27 | 27 | 28 | 27 | | |
| (g/h) | | | | | | | | |
| Zusammensetzung | | | | | | | | |
| (Gew.-%) | | | | | | | | |
| C₆ | 99,9 | 68,5 | 52,7 | 43,6 | 57,0 | 73,2 | n.e. | 0,1 |
| C₇-C₁₁ | 0,1 | 0,2 | 0,2 | 0,3 | 0,2 | 0,2 | | - |
| C₁₂ | | 25,9 | 38,6 | 44,0 | 35,6 | 23,6 | | 99,9 |
| C₁₃+ | | 5,4 | 8,5 | 12,1 | 7,2 | 3,0 | | - |
| | | | | | | | | |
| Umsatz | | 31,4 | 47,2 | 56,4 | 42,9 | 26,7 | | |
| C12-Selektivität | | 82,5 | 81,8 | 78,2 | 83,0 | 88,4 | | |
| (Gew.-%) | | | | | | | | |
| S-Gehalt im | < 1 | n.e. | n.e. | n.e. | n.e. | n.e. | n.e. | n.e. |
| Flüssiganfall | | | | | | | | |
| (ppm) | | | | | | | | |

Das Sammel-Produkt wurde bis zu einer C₁₂-Reinheit von 99,9 Gew.-% aufdestilliert.

### Analytik:

Zu den Proben wird n-Oktan und n-Hexadecan zugewogen, die Bestimmung der Ausbeute erfolgt via GC-FID (Säule 50m DB-5, 0,1 µm-Film) nach der Methode des internen Standards (Benzol bezogen auf Oktan, MLAB auf Hexadecan). Die Bestimmung des unumgesetzten Olefins erfolgt via GC-MS relativ zu n-Oktan als internem Standard. Der absolute Fehler liegt bei ca. 3% absolut.

### Vergleichsbeispiel 1- ein Reaktor:

Verwendet wurde ein Reaktor mit 70g Katalysator. Das Benzol: Dodecen - Verhältnis betrug 5 mol: 1 mol, die Feedbelastung 0,42 g Feed / g Katalysator / h, d.h. 29,4g / h. Es wurden folgende Umsätze und Ausbeuten gemessen:

| Laufzeit [h] | Olefin | | Benzol | |
|---|---|---|---|---|
| | Ausbeute [mol-%] | Umsatz [mol-%] | Ausbeute [mol-%] | Umsatz [mol-%] |
| 19 | 80% | 100% | 16% | 24% |
| 26 | 83% | 100% | 17% | 25% |
| 42,5 | 84% | 100% | 17% | 25% |
| 50 | 84% | 100% | 17% | 24% |
| 67 | 83% | 100% | 17% | 24% |
| 73,5 | 85% | 100% | 17% | 25% |
| 95,5 | 88% | 100% | 18% | 25% |
| 120 | 87% | 100% | 17% | 24% |
| 139 | 89% | 100% | 18% | 23% |
| 146 | 88% | 100% | 17% | 23% |
| 163 | 83% | 100% | 17% | 22% |
| 170 | 76% | 98% | 15% | 21% |
| 187 | 42% | 50% | 8% | 13% |
| 194 | 39% | 42% | 8% | 18% |

### Ergebnis:

Nach ca. 160 Stunden war der Katalysator so weit deaktiviert, daß kein Vollumsatz mehr erreicht werden konnte.

### Beispiel 2 - Kaskade aus 3 Reaktoren:

Verwendet wurden drei Reaktoren mit je 70g Katalysator. Der erste Reaktor wurde mit einem Gemisch im Verhältnis Benzol : Dodecen = 15 mol : 1 mol und einer Belastung von 0,336 g / g / h belastet. Der zweite Reaktor erhält den Produktsrom des ersten Reaktors plus 0,042 g / g / h Dodecen. Der dritte Reaktor erhält den Produktstrom des zweiten Reaktors plus 0,042 g / g / h Dodecen. Dies entspricht einer Gesamtbelastung von 0,42 g / g / h bei einem globalen Feedverhältnis von 5 mol : 1 mol. Nach 65 h Laufzweit wurde über Ventile der Hauptfeedstrom von der Reihenfolge 1-2-3 auf die Reihenfolge 2-3-1, nach 130 h von 2-3-1- auf 3-1-2 und nach 195 h von 3-1-2- auf 1-2-3 geschaltete. Es wurden folgende Umsätze und Ausbeuten über die gesamte Kaskade gemessen:

| Laufzeit [h] | Olefin | | Benzol | |
|---|---|---|---|---|
| | Ausbeute [mol-%] | Umsatz [mol-%] | Ausbeute [mol-%] | Umsatz [mol-%] |
| 20 | 81 % | 100% | 16% | 24% |
| 28 | 85% | 100% | 17% | 25% |
| 50 | 86% | 100% | 17% | 24% |
| 65,5 | 85% | 100% | 17% | 24% |
| 72,5 | 86% | 100% | 17% | 25% |
| 94,5 | 90% | 100% | 18% | 25% |
| 117 | 89% | 100% | 18% | 24% |
| 141 | 91% | 100% | 18% | 23% |
| 147 | 89% | 100% | 17% | 23% |
| 161 | 90% | 100% | 17% | 24% |
| 168 | 89% | 100% | 19% | 25% |
| 186 | 91 % | 100% | 18% | 22% |
| 192 | 90% | 100% | 18% | 23% |
| 199 | 85% | 98% | 17% | 22% |
| 214 | 54% | 60% | 10% | 14% |
| 230,5 | 20% | 22% | 5% | 7% |

### Ergebnis:

Neben einer Erhöhung der Selektivität bezüglich Dodecen um ca. 2 Prozentpunkte war eine um 22% erhöhte Katalysatorstandzeit (199 statt 163 h) relativ zu einem Reaktor zu verzeichnen.

## Patentansprüche

1. Verfahren zur Herstellung von alkylaromatischen Verbindungen durch Umsetzung von C₃₋₃₀-Olefinen oder Alkoholen, aus denen bei den Umsetzungsbedingungen C₃₋₃₀-Olefine gebildet werden, mit einem aromatischen Kohlenwasserstoff in Gegenwart eines Alkylierungskatalysators, **dadurch gekennzeichnet, dass** die Umsetzung in einer Reaktorkaskade aus mindestens zwei Reaktoren durchgeführt wird, wobei jeder der Reaktoren den Alkylierungskatalysator enthält, mindestens 80 % des aromatischen Kohlenwasserstoffs in den ersten Reaktor der Reaktorkaskade eingespeist werden und mindestens 40 % der Olefine nach dem ersten Reaktor zwischeneingespeist werden.

2. Verfahren nach Anspruch 1, **dadurch gekennzeichnet, dass** die Reaktorkaskade mindestens drei Reaktoren aufweist und eine Zwischeneinspeisung des Olefins vor jedem der Reaktoren durchgeführt wird.

3. Verfahren nach Anspruch 1 oder 2, **dadurch gekennzeichnet, dass** jeweils gleiche Anteile des Olefins in jeden Reaktor eingespeist werden.

4. Verfahren nach Anspruch 1 oder 2, **dadurch gekennzeichnet, dass** eine Zwischeneinspeisung des Olefins vor jedem der Reaktoren durchgeführt wird und die jeweils zwischeneingespeiste Olefinmenge so gesteuert wird, dass in jedem Reaktor, bezogen auf die jeweilige Katalysatormenge, die gleiche inkrementelle Produktivität erreicht wird.

5. Verfahren nach einem der Ansprüche 1 bis 4, **dadurch gekennzeichnet, dass** die Reihenfolge der Reaktoren innerhalb der Kaskade in zeitlichen Abständen so geändert wird, dass jeder Reaktor jede der Positionen innerhalb der Kaskade für den gleichen Zeitraum einnimmt.

6. Verfahren nach einem der Ansprüche 1 bis 5, **dadurch gekennzeichnet, dass** die Reaktoren in der Kaskade jeweils eine Rührkesselcharakteristik aufweisen.

7. Verfahren nach einem der Ansprüche 1 bis 6, **dadurch gekennzeichnet, dass** die Alkylierung in der Flüssigphase bei Temperaturen im Bereich von 100 bis 250 °C durchgeführt wird.

8. Verfahren nach einem der Ansprüche 1 bis 7, **dadurch gekennzeichnet, dass** der Alkylierungskatalysator ein Heterogenkatalysator ist, ausgewählt aus sauren Tonen, sauren Ionentauschern, sauren Metalloxiden, sulfatisierten Metalloxiden, geträgerten Heteropolysäuren und Zeolithen.

9. Verfahren nach einem der Ansprüche 1 bis 8, **dadurch gekennzeichnet, dass** C₁₀₋₁₂-Olefine eingesetzt werden, die einen mittleren Verzweigungsgrad von mindestens 0,5 aufweisen.

10. Verfahren nach Anspruch 9, **dadurch gekennzeichnet, dass** die C₁₀₋₁₂-Olefine erhalten werden durch
Umsetzung eines C₄-Olefin-Gemisches an einem Metathese-Katalysator zur Herstellung einer 2-Penten und/oder 3-Hexen enthaltenden Olefingemisches und gegebenenfalls Abtrennung von 2-Penten und/oder 3-Hexen, und
Dimerisierung der so erhaltenen 2-Pentens und/oder 3-Hexens in Gegenwart eines Dimerisierungskatalysators zu einem C₁₀₋₁₂-Olefine enthaltenden Gemisch, gegebenenfalls gefolgt von Abtrennung der C₁₀₋₁₂-Olefine und Abtrennung von 5 bis 30 Gew.-%, bezogen auf die abgetrennten C₁₀₋₁₂-Olefine, an Leichtsiederbestandteilen der C₁₀₋₁₂-Olefine.

11. Verfahren zur Herstellung von Alkylarylsulfonaten durch
a) Umsetzung eines C₄-Olefin-Gemisches an einem Metathesekatalysator zur Herstellung eines 2-Penten und/oder 3-Hexen enthaltenden Olefingemisches und gegebenenfalls Abtrennung von 2-Penten und/oder 3-Hexen,
b) Dimerisierung des in Stufe a) erhaltenen 2-Pentens und/oder 3-Hexens in Gegenwart eines Dimerisierungskatalysators zu einem C₁₀₋₁₂-Olefine enthaltenden Gemisch, gegebenenfalls Abtrennung der C₁₀₋₁₂-Olefine und Abtrennung von 5 bis 30 Gew.-%, bezogen auf die abgetrennten C₁₀₋₁₂-Olefine, an Leichtsieder-Bestandteilen der C₁₀₋₁₂-Olefine,
c) Umsetzung der in Stufe b) erhaltenen C₁₀₋₁₂-Olefin-Gemische mit einem aromatischen Kohlenwasserstoff in Gegenwart eines Alkylierungskatalysators zur Bildung von alkylaromatischen Verbindungen, wobei vor der Umsetzung zusätzlich 0 bis 60 Gew.-%, bezogen auf die in Stufe b) erhaltenen C₁₀₋₁₂-Olefin-Gemische, an linearen Olefinen zugesetzt werden können,
d) Sulfonierung der in Stufe c) erhaltenen alkylaromatischen Verbindungen und Neutralisation zu Alkylarylsulfonaten, wobei vor der Sulfonierung zusätzlich 0 bis 60 Gew.-%, bezogen auf die in Stufe c) erhaltenen alkylaromatischen Verbindungen, an linearen Alkylbenzolen zugesetzt werden können, sofern keine Zumischung in Stufe c) erfolgt ist,
e) gegebenenfalls Abmischen der in Stufe d) erhaltenen Alkylarylsulfonate mit 0 bis 60 Gew.-%, bezogen auf die in Stufe d) erhaltenen Alkylarylsulfonate, an linearen Alkylarylsulfonaten, sofern keine Zumischungen in Stufen c) und d) erfolgt sind,
**dadurch gekennzeichnet, dass** die Umsetzung in Stufe c) nach einem Verfahren gemäß einem der Ansprüche 1 bis 8 durchgeführt wird.

## Claims

1. The process for the preparation of alkylaromatic compounds by reacting C₃₋₃₀-olefins, or alcohols from which C₃₋₃₀-olefins are formed under the reaction conditions, with an aromatic hydrocarbon in the presence of an alkylation catalyst, which comprises carrying out the reaction in a reactor cascade of at least two reactors, where each of the reactors comprises the alkylation catalyst, at least 80% of the aromatic hydrocarbon are fed into the first reactor of the reactor cascade, and at least 40% of the olefins are intermediately fed in after the first reactor.

2. The process according to claim 1, wherein the reactor cascade has at least three reactors, and the olefin is intermediately fed in before each of the reactors.

3. The process according to claim 1 or 2, wherein in each case equal proportions of the olefin are fed into each reactor.

4. The process according to claim 1 or 2, wherein the olefin is intermediately fed in before each of the reactors and the amount of olefin intermediately fed in in each case is controlled such that, in each reactor, the same incremental productivity is achieved, based on the respective amount of catalyst.

5. The process according to any of claims 1 to 4, wherein the order of the reactors within the cascade is changed at time intervals such that each reactor assumes each of the positions within the cascade for the same period of time.

6. The process according to any of claims 1 to 5, wherein the reactors in the cascade each have the characteristics of a stirred-tank reactor.

7. The process according to any of claims 1 to 6, wherein the alkylation is carried out in the liquid phase at temperatures in the range from 100 to 250°C.

8. The process according to any of claims 1 to 7, wherein the alkylation catalyst is a heterogeneous catalyst chosen from acidic clays, acidic ion exchangers, acidic metal oxides, sulfated metal oxides, supported heteropolyacids and zeolites.

9. The process according to any of claims 1 to 8, wherein C₁₀₋₁₂-olefins are used which have an average degree of branching of at least 0.5.

10. The process according to claim 9, wherein the C₁₀₋₁₂-olefins are obtained by
reacting a C₄-olefin mixture over a metathesis catalyst to prepare an olefin mixture comprising 2-pentene and/or 3-hexene, and, if appropriate, separating off 2-pentene and/or 3-hexene, and
dimerizing the resulting 2-pentene and/or 3-hexene in the presence of a dimerization catalyst to give a mixture comprising C₁₀₋₁₂-olefins, if appropriate followed by separating off the C₁₀₋₁₂-olefins and separating off 5 to 30% by weight, based on the separated-off C₁₀₋₁₂-olefins, of low-boiling constituents of the C₁₀₋₁₂-olefins.

11. A process for the preparation of alkylarylsulfonates by
a) reacting a C₄-olefin mixture over a metathesis catalyst to prepare an olefin mixture comprising 2-pentene and/or 3-hexene, and, if appropriate, separating off 2-pentene and/or 3-hexene,
b) dimerizing the 2-pentene and/or 3-hexene obtained in stage a) in the presence of a dimerization catalyst to give a mixture comprising C₁₀₋₁₂-olefins, if appropriate separating off the C₁₀₋₁₂-olefins and separating off 5 to 30% by weight, based on the separated-off C₁₀₋₁₂-olefins, of low-boiling constituents of the C₁₀₋₁₂-olefins,
c) reacting the C₁₀₋₁₂-olefin mixtures obtained in stage b) with an aromatic hydrocarbon in the presence of an alkylation catalyst to form alkylaromatic compounds, where, prior to the reaction, 0 to 60% by weight, based on the C₁₀₋₁₂-olefin mixtures obtained in stage b), of linear olefins may additionally be added,
d) sulfonating the alkylaromatic compounds obtained in stage c) and neutralizing them to give alkylarylsulfonates, where, prior to the sulfonation, 0 to 60% by weight, based on the alkylaromatic compounds obtained in stage c), of linear alkylbenzenes may additionally be added if no such addition has taken place in stage c),
e) if appropriate mixing the alkylarylsulfonates obtained in stage d) with 0 to 60% by weight, based on the alkylarylsulfonates obtained in stage d), of linear alkylarylsulfonates, if no such additions have taken place in stages c) and d),
wherein the reaction in stage c) is carried out in accordance with a process according to any of claims 1 to 8.

## Revendications

1. Procédé de fabrication de composés alkyle aromatiques grâce à la réaction d'oléfines en C₃₋₃₀ ou d'alcools, à partir desquels des oléfines en C₃-30 sont formées dans des conditions de réaction, avec un hydrocarbure aromatique en présence d'un catalyseur d'alkylation, **caractérisé en ce que** la réaction a lieu dans une cascade de réacteurs constituée d'au moins deux réacteurs, chacun des réacteurs contenant le catalyseur d'alkylation, au moins 80 % de l'hydrocarbure aromatique étant stocké dans le premier réacteur de la cascade de réacteurs et au moins 40 % des oléfines étant stockées en position intermédiaire après le premier réacteur.

2. Procédé selon la revendication 1, **caractérisé en ce que** la cascade de réacteurs présente au moins trois réacteurs et qu'un stockage intermédiaire de l'oléfine est réalisé avant chacun des réacteurs.

3. Procédé selon l'une quelconque des revendications 1 ou 2, **caractérisé en ce que** des quantités identiques d'oléfine sont stockées dans chaque réacteur.

4. Procédé selon la revendication 1 ou 2, **caractérisé en ce qu'**un stockage intermédiaire de l'oléfine est réalisé avant chaque réacteur et que la quantité d'oléfine stockée de manière intermédiaire est gérée de façon à ce que, dans chaque réacteur, par rapport à la quantité de catalyseur respective, on obtienne la même productivité incrémentielle.

5. Procédé selon l'une quelconque des revendications 1 à 4, **caractérisé en ce que** l'ordre des réacteurs au sein de la cascade est modifié de telle façon dans les intervalles de temps que chaque réacteur occupe chacune des positions au sein de la cascade pendant la même durée.

6. Procédé selon l'une quelconque des revendications 1 à 5, **caractérisé en ce que** les réacteurs de la cascade présentent chacun comme caractéristique un récipient agité.

7. Procédé selon l'une quelconque des revendications 1 à 6, **caractérisé en ce que** l'alkylation de la phase liquide est réalisée à des températures situées dans la plage allant de 100 à 250°C.

8. Procédé selon l'une quelconque des revendications 1 à 7, **caractérisé en ce que** le catalyseur de l'alkylation est un catalyseur hétérogène, choisi parmi les argiles acides, les échangeurs d'ions acides, les oxydes de métaux acides, les oxydes de métaux sulfatés, les hétéropolyacides sur substrat et les zéolithes.

9. Procédé selon l'une quelconque des revendications 1 à 8, **caractérisé en ce que** des oléfines en C₁₀₋₁₂ sont utilisées qui présentent un degré de ramification moyen d'au moins 0,5.

10. Procédé selon la revendication 9, **caractérisé en ce que** les oléfines en C₁₀₋₁₂ sont obtenues grâce à la réaction d'un mélange d'oléfines en C₄ avec un catalyseur de métathèse pour la fabrication d'un mélange oléfinique contenant un 2-pentène et/ou un 3-hexène et le cas échéant l'isolement du 2-pentène et/ou du 3-hexène, et
à la dimérisation du 2-pentène et/ou du 3-hexène ainsi obtenus en présence d'un catalyseur de dimérisation pour donner un mélange contenant des oléfines en C₁₀₋₁₂, le cas échéant suivie d'un isolement des oléfines en C₁₀₋₁₂ et l'isolement de 5 à 30 % en poids, par rapport aux oléfines en C₁₀₋₁₂ isolées, de composants à bas point d'ébullition des oléfines en C₁₀₋₁₂.

11. Procédé pour la fabrication de sulfonates d'alkylaryle grâce à :
a) la réaction d'un mélange d'oléfines en C₄ avec un catalyseur de métathèse pour la fabrication d'un mélange oléfinique contenant du 2-pentène et/ou du 3-hexène et le cas échéant l'isolement du 2-pentène et/ou du 3-hexène,
b) la dimérisation du 2-pentène et/ou 3-hexène obtenus à l'étape a) en présence du catalyseur de dimérisation pour donner un mélange contenant des oléfines en C₁₀₋₁₂, éventuellement l'isolement des oléfines en C₁₀₋₁₂ et l'isolement de 5 à 30 % en poids, par rapport aux oléfines en C₁₀₋₁₂ isolées, de composants à bas point d'ébullition des oléfines en C₁₀₋₁₂,
c) la réaction du mélange d'oléfines en C₁₀₋₁₂ obtenu à l'étape b) avec un hydrocarbure aromatique en présence d'un catalyseur d'alkylation pour la formation de composés alkyle aromatiques, 0 à 60 % en poids supplémentaires d'oléfines linéaires, par rapport au mélange d'oléfines en C₁₀₋₁₂ obtenu à l'étape b), pouvant être ajoutés avant la réaction,
d) la sulfonation des composés alkyle aromatiques obtenus à l'étape c) et la neutralisation en sulfonates d'alkylaryle, 0 à 60 % en poids supplémentaires d'alkylbenzoles linéaires, par rapport au composés alkyle aromatiques obtenus à l'étape c), pouvant être ajoutés avant la sulfonation, tant qu'aucun mélange ne se produit à l'étape c),
e) le cas échéant le mélange des sulfonates d'alkylaryle obtenus à l'étape d) avec de 0 à 60 % en poids de sulfonates d'alkylaryle linéaires, par rapport aux sulfonates d'alkylaryle obtenus à l'étape d), tant qu'aucun mélange ne s'est produit aux étapes c) et d),
**caractérisé en ce que** la réaction de l'étape c) est réalisée conformément à un procédé selon l'une quelconque des revendications 1 à 8.
